# EUROPEAN PATENT SPECIFICATION

(11) **EP 1 731 166 B1**
(45) Date of publication and mention of the grant of the patent: **09.03.2011**
(21) Application number: 06009322.6
(22) Date of filing: 09.06.2000
(51) Int. Cl.: A61K 39/09, C07K 16/12, C12N 5/10, A61K 39/40, A61P 31/04, C07K 14/315

(54) **Streptococcus pneumoniae proteins and vaccines**
Streptococcus pneumoniae Proteine und Impfstoffe
Protéines et vaccins de streptococcus pneumoniae

(30) Priority: 10.06.1999 US 138453 P
(43) Date of publication of application: 13.12.2006
(62) Divisional of application: 00939739.9
(73) Proprietor: MedImmune, LLC, Gaithersburg, MD 20878 (US); HUMAN GENOME SCIENCES, INC., Rockville, MD 20850 (US)
(72) Inventor: Adamou, John E., New Milford, CT 06776 (US); Choi, Gil H., Rockville, MD 20850 (US)
(74) Representative: Chapman, Paul Gilmour

(56) References cited:
- WO-A-00/06738
- WO-A-98/18930
- NAYAK A R ET AL: "A LIVE RECOMBINANT AVIRULENT ORAL SALMONELLA VACCINE EXPRESSING PNEUMOCOCCAL SURFACE PROTEIN A INDUCES PROTECTIVE RESPONSES AGAINSTSTREPTOCOCCUS PNEUMONIAE" INFECTION AND IMMUNITY,AMERICAN SOCIETY FOR MICROBIOLOGY. WASHINGTON,US, vol. 66, August 1998 (1998-08), pages 3744-3751, XP000918253 ISSN: 0019-9567

### SEQUENCE LISTING

<110> Adamou, John
   Choi, Gil
<120> Streptococcus Pneumoniae Proteins and Vaccines
<130> 469201-475
<140>
   <141>
<150> U.S. 60/138,453 <151> 1999-06-10
<160> 8
<170> Patent In Ver. 2.1
<210> 1
   <211> 21
   <212> DNA
   <213> Artificial Sequence
<220>
   <223> Description of Artificial Sequence:Forward primer for PCR amplification of Sp128 genomic sequences
<400> 1
   tacccggtag tcttagcaga c 21
<210> 2
   <211> 25
   <212> DNA
   <213> Artificial Sequence
<220>
   <223> Description of Artificial Sequence:Reverse primer for PCR amplification of Sp128 genomic sequence
<400> 2
   atagccataa gttgatttgc catta 25
<210> 3
   <211> 21
   <212> DNA
   <213> Artificial Sequence
<220>
   <223> Description of Artificial Sequence:Forward primer for PCR amplification of Sp130 genomic sequence
<400> 3
   aagcttggcg agattgcaga a 21
<210> 4
   <211> 27
   <212> DNA
   <213> Artificial Sequence
<220>
   <223> Description of Artificial Sequence:Reverse primer for PCR amplification of Sp130 genomic sequence
<400> 4
   cttattagga ttgttagtag ttgattt 27
<210> 5
   <211> 1992
   <212> DNA
   <213> Streptococcus pneumoniae
<400> 5
   tacccggtag tcttagcaga cacatctagc tctgaagatg ctttaaacat ctctgataaa 60
   gaaaaagtag cagaaaataa agagaaacat gaaaatatcc atagtgctat ggaaacttca 120
   caggatttta aagagaagaa aacagcagtc attaaggaaa aagaagttgt tagtaaaaat 180
   cctgtgatag acaataacac tagcaatgaa gaagcaaaaa tcaaagaaga aaattccaat 240
   aaatcccaag gagattatac ggactcattt gtgaataaaa acacagaaaa tcccaaaaaa 300
   gaagataaag ttgtctatat tgctgaattt aaagataaag aatctggaga aaaagcaatc 360
   aaggaactat ccagtcttaa gaatacaaaa gttttatata cttatgatag aatttttaac 420
   ggtagtgcca tagaaacaac tccagataac ttggacaaaa ttaaacaaat agaaggtatt 480
   tcatcggttg aaagggcaca aaaagtccaa cccatgatga atcatgccag aaaggaaatt 540
   ggagttgagg aagctattga ttacctaaag tctatcaatg ctccgtttgg gaaaaatttt 600
   gatggtagag gtatggtcat ttcaaatatc gatactggaa cagattatag acataaggct 660
   atgagaatcg atgatgatgc caaagcctca atgagattta aaaaagaaga cttaaaaggc 720
   actgataaaa attattggtt gagtgataaa atccctcatg cgttcaatta ttataatggt 780
   ggcaaaatca ctgtagaaaa atatgatgat ggaagggatt attttgaccc acatgggatg 840
   catattgcag ggattcttgc tggaaatgat actgaacaag acatcaaaaa ctttaacggc 900
   atagatggaa ttgcacctaa tgcacaaatt ttctcttaca aaatgtattc tgacgcagga 960
   tctgggtttg cgggtgatga aacaatgttt catgctattg aagattctat caaacacaac 1020
   gttgatgttg tttcggtatc atctggtttt acaggaacag gtcttgtagg tgagaaatat 1080
   tggcaagcta ttcgggcatt aagaaaagca ggcattccaa tggttgtcgc tacgggtaac 1140
   tatgcgactt ctgcttcaag ttcttcatgg gatttagtag caaataatca tctgaaaatg 1200
   accgacactg gaaatgtaac acgaactgca gcacatgaag atgcgatagc ggtcgcttct 1260
   gctaaaaatc aaacagttga gtttgataaa gttaacatag gtggagaaag ttttaaatac 1320
   agaaatatag ggcctttttt cgataagagt aaaatcacaa caaatgaaga tggaacaaaa 1380
   gctcctagta aattaaaatt tgtatatata ggcaaggggc aagaccaaga tttgataggt 1440
   ttggatctta ggggcaaaat tgcagtaatg gatagaattt atacaaagga tttaaaaaat 1500
   gcttttaaaa aagctatgga taagggtgca cgcgccatta tggttgtaaa tactgtaaat 1560
   tactacaata gagataattg gacagagctt ccagctatgg gatatgaagc ggatgaaggt 1620
   actaaaagtc aagtgttttc aatttcagga gatgatggtg taaagctatg gaacatgatt 1680
   aatcctgata aaaaaactga agtcaaaaga aataataaag aagattttaa agataaattg 1740
   gagcaatact atccaattga tatggaaagt tttaattcca acaaaccgaa tgtaggtgac 1800
   gaaaaagaga ttgactttaa gtttgcacct gacacagaca aagaactcta taaagaagat 1860
   atcatcgttc cagcaggatc tacatcttgg gggccaagaa tagatttact tttaaaaccc 1920
   gatgtttcag cacctggtaa aaatattaaa tccacgctta atgttattaa tggcaaatca 1980
   acttatggct at                                                      1992
<210> 6
   <211> 664
   <212> PRT
   <213> Streptococcus pneumoniae
<400> 6
   Tyr Pro Val Val Leu Ala Asp Thr Ser Ser Ser Glu Asp Ala Leu Asn
   1 5 10 15
   Ile Ser Asp Lys Glu Lys Val Ala Glu Asn Lys Glu Lys His Glu Asn
   20 25 30
   Ile His Ser Ala Met Glu Thr Ser Gln Asp Phe Lys Glu Lys Lys Thr
   35 40 45
   Ala Val Ile Lys Glu Lys Glu Val Val Ser Lys Asn Pro Val Ile Asp
   50 55 60
   Asn Asn Thr Ser Asn Glu Glu Ala Lys Ile Lys Glu Glu Asn Ser Asn
   65 70 75 80
   Lys Ser Gln Gly Asp Tyr Thr Asp Ser Phe Val Asn Lys Asn Thr Glu
   85 90 95
   Asn Pro Lys Lys Glu Asp Lys Val Val Tyr Ile Ala Glu Phe Lys Asp
   100 105 110
   Lys Glu Ser Gly Glu Lys Ala Ile Lys Glu Leu Ser Ser Leu Lys Asn
   115 120 125
   Thr Lys Val Leu Tyr Thr Tyr Asp Arg Ile Phe Asn Gly Ser Ala Ile
   130 135 140
   Glu Thr Thr Pro Asp Asn Leu Asp Lys Ile Lys Gln Ile Glu Gly Ile
   145 150 155 160
   Ser Ser Val Glu Arg Ala Gln Lys Val Gln Pro Met Met Asn His Ala
   165 170 175
   Arg Lys Glu Ile Gly Val Glu Glu Ala Ile Asp Tyr Leu Lys Ser Ile
   180 185 190
   Asn Ala Pro Phe Gly Lys Asn Phe Asp Gly Arg Gly Met Val Ile Ser
   195 200 205
   Asn Ile Asp Thr Gly Thr Asp Tyr Arg His Lys Ala Met Arg Ile Asp
   210 215 220
   Asp Asp Ala Lys Ala Ser Met Arg Phe Lys Lys Glu Asp Leu Lys Gly
   225 230 235 240
   Thr Asp Lys Asn Tyr Trp Leu Ser Asp Lys Ile Pro His Ala Phe Asn
   245 250 255
   Tyr Tyr Asn Gly Gly Lys Ile Thr Val Glu Lys Tyr Asp Asp Gly Arg
   260 265 270
   Asp Tyr Phe Asp Pro His Gly Met His Ile Ala Gly Ile Leu Ala Gly
   275 280 285
   Asn Asp Thr Glu Gln Asp Ile Lys Asn Phe Asn Gly Ile Asp Gly Ile
   290 295 300
   Ala Pro Asn Ala Gln Ile Phe Ser Tyr Lys Met Tyr Ser Asp Ala Gly
   305 310 315 320
   Ser Gly Phe Ala Gly Asp Glu Thr Met Phe His Ala Ile Glu Asp Ser
   325 330 335
   Ile Lys His Asn Val Asp Val Val Ser Val Ser Ser Gly Phe Thr Gly
   340 345 350
   Thr Gly Leu Val Gly Glu Lys Tyr Trp Gln Ala Ile Arg Ala Leu Arg
   355 360 365
   Lys Ala Gly Ile Pro Met Val Val Ala Thr Gly Asn Tyr Ala Thr Ser
   370 375 380
   Ala Ser Ser Ser Ser Trp Asp Leu Val Ala Asn Asn His Leu Lys Met
   385 390 395 400
   Thr Asp Thr Gly Asn Val Thr Arg Thr Ala Ala His Glu Asp Ala Ile
   405 410 415
   Ala Val Ala Ser Ala Lys Asn Gln Thr Val Glu Phe Asp Lys Val Asn
   420 425 430
   Ile Gly Gly Glu Ser Phe Lys Tyr Arg Asn Ile Gly Ala Phe Phe Asp
   435 440 445
   Lys Ser Lys Ile Thr Thr Asn Glu Asp Gly Thr Lys Ala Pro Ser Lys
   450 455 460
   Leu Lys Phe Val Tyr Ile Gly Lys Gly Gln Asp Gln Asp Leu Ile Gly
   465 470 475 480
   Leu Asp Leu Arg Gly Lys Ile Ala Val Met Asp Arg Ile Tyr Thr Lys
   485 490 495
   Asp Leu Lys Asn Ala Phe Lys Lys Ala Met Asp Lys Gly Ala Arg Ala
   500 505 510
   Ile Met Val Val Asn Thr Val Asn Tyr Tyr Asn Arg Asp Asn Trp Thr
   515 520 525
   Glu Leu Pro Ala Met Gly Tyr Glu Ala Asp Glu Gly Thr Lys Ser Gln
   530 535 540
   Val Phe Ser Ile Ser Gly Asp Asp Gly Val Lys Leu Trp Asn Met Ile
   545 550 555 560
   Asn Pro Asp Lys Lys Thr Glu Val Lys Arg Asn Asn Lys Glu Asp Phe
   565 570 575
   Lys Asp Lys Leu Glu Gln Tyr Tyr Pro Ile Asp Met Glu Ser Phe Asn
   580 585 590
   Ser Asn Lys Pro Asn Val Gly Asp Glu Lys Glu Ile Asp Phe Lys Phe
   595 600 605
   Ala Pro Asp Thr Asp Lys Glu Leu Tyr Lys Glu Asp Ile Ile Val Pro
   610 615 620
   Ala Gly Ser Thr Ser Trp Gly Pro Arg Ile Asp Leu Leu Leu Lys Pro
   625 630 635 640
   Asp Val Ser Ala Pro Gly Lys Asn Ile Lys Ser Thr Leu Asn Val Ile
   645 650 655
   Asn Gly Lys Ser Thr Tyr Gly Tyr
   660
<210> 7
   <211> 2319
   <212> DNA
   <213> Streptococcus pneumoniae
<400> 7
   aagcttggcg agattgcaga atctaaattt aaaaatttag gaaatggaaa agagggtagt 60
   ctaaaaaaag atacaactgg ggtagaacat catcatcaag aaaatgaaga gtctattaaa 120
   gaaaaatcta gttttactat tgatagaaat atttcaacaa ttagagactt tgaaaataaa 180
   gacttaaaga aactcattaa aaagaaattt agagaagttg atgattttac aagtgaaact 240
   ggtaagagaa tggaggaata cgattataaa tacgatgata aaggaaatat aatagcctac 300
   gatgatggga ctgatctaga atatgaaact gagaaacttg acgaaatcaa atcaaaaatt 360
   tatggtgttc taagtccgtc taaagatgga cactttgaaa ttcttggaaa gataagtaat 420
   gtttctaaaa atgccaaggt atattatggg aataactata aatctataga aatcaaagcg 480
   accaagtatg atttccactc aaaaacgatg acatttgatc tatacgctaa tattaatgat 540
   attgtggatg gattagcttt tgcaggagat atgagattat ttgttaaaga taatgatcag 600
   aaaaaagctg aaattaaaat tagaatgcct gaaaaaatta aggaaactaa atcagaatat 660
   ccctatgtat caagttatgg gaatgtcata gaattagggg aaggagatct ttcaaaaaac 720
   aaaccagaca atttaactaa aatggaatct ggtaaaatct attctgattc agaaaaacaa 780
   caatatctgt taaaggataa tatcattcta agaaaaggct atgcactaaa agtgactacc 840
   tataatcctg gaaaaacgga tatgttagaa ggaaatggag tctatagcaa ggaagatata 900
   gcaaaaatac aaaaggccaa tcctaatcta agagcccttt cagaaacaac aatttatgct 960
   gatagtagaa atgttgaaga tggaagaagt acccaatctg tattaatgtc ggctttggac 1020
   ggctttaaca ttataaggta tcaagtgttt acatttaaaa tgaacgataa aggggaagct 1080
   atcgataaag acggaaatct tgtgacagat tcttctaaac ttgtattatt tggtaaggat 1140
   gataaagaat acactggaga ggataagttc aatgtagaag ctataaaaga agatggctcc 1200
   atgttattta ttgataccaa accagtaaac ctttcaatgg ataagaacta ctttaatcca 1260
   tctaaatcta ataaaattta tgtacgaaat ccagaatttt atttaagagg taagatttct 1320
   gataagggtg gttttaactg ggaattgaga gttaatgaat cggttgtaga taattattta 1380
   atctacggag atttacacat tgataacact agagatttta atattaagct gaatgttaaa 1440
   gacggtgaca tcatggactg gggaatgaaa gactataaag caaacggatt tccagataag 1500
   gtaacagata tggatggaaa tgtttatctt caaactggct atagcgattt gaatgctaaa 1560
   gcagttggag tccactatca gtttttatat gataatgtta aacccgaagt aaacattgat 1620
   cctaagggaa atactagtat cgaatatgct gatggaaaat ctgtagtctt taacatcaat 1680
   gataaaagaa ataatggatt cgatggtgag attcaagaac aacatattta tataaatgga 1740
   aaagaatata catcatttaa tgatattaaa caaataatag acaagacact aaacattaag 1800
   attgttgtaa aagattttgc aagaaataca accgtaaaag aattcatttt aaataaagat 1860
   acgggagagg taagtgaatt aaaacctcat agggtaactg tgaccattca aaatggaaaa 1920
   gaaatgagtt caacgatagt gtcggaagaa gattttattt tacctgttta taagggtgaa 1980
   ttagaaaaag gataccaatt tgatggttgg gaaatttctg gtttcgaagg taaaaaagac 2040
   gctggctatg ttattaatct atcaaaagat acctttataa aacctgtatt caagaaaata 2100
   gaggagaaaa aggaggaaga aaataaacct acttttgatg tatcgaaaaa gaaagataac 2160
   ccacaagtaa accatagtca attaaatgaa agtcacagaa aagaggattt acaaagagaa 2220
   gagcattcac aaaaatctga ttcaactaag gatgttacag ctacagttct tgataaaaac 2280
   aatatcagta gtaaatcaac tactaacaat cctaataag 2319
<210> 8
   <211> 773
   <212> PRT
   <213> Streptococcus pneumoniae
<400> 8
   Lys Leu Gly Glu Ile Ala Glu Ser Lys Phe Lys Asn Leu Gly Asn Gly
   1 5 10 15
   Lys Glu Gly Ser Leu Lys Lys Asp Thr Thr Gly Val Glu His His His
   20 25 30
   Gln Glu Asn Glu Glu Ser Ile Lys Glu Lys Ser Ser Phe Thr Ile Asp
   35 40 45
   Arg Asn Ile Ser Thr Ile Arg Asp Phe Glu Asn Lys Asp Leu Lys Lys
   50 55 60
   Leu Ile Lys Lys Lys Phe Arg Glu Val Asp Asp Phe Thr Ser Glu Thr
   65 70 75 80
   Gly Lys Arg Met Glu Glu Tyr Asp Tyr Lys Tyr Asp Asp Lys Gly Asn
   85 90 95
   Ile Ile Ala Tyr Asp Asp Gly Thr Asp Leu Glu Tyr Glu Thr Glu Lys
   100 105 110
   Leu Asp Glu Ile Lys Ser Lys Ile Tyr Gly Val Leu Ser Pro Ser Lys
   115 120 125
   Asp Gly His Phe Glu Ile Leu Gly Lys Ile Ser Asn Val Ser Lys Asn
   130 135 140
   Ala Lys Val Tyr Tyr Gly Asn Asn Tyr Lys Ser Ile Glu Ile Lys Ala
   145 150 155 160
   Thr Lys Tyr Asp Phe His Ser Lys Thr Met Thr Phe Asp Leu Tyr Ala
   165 170 175
   Asn Ile Asn Asp Ile Val Asp Gly Leu Ala Phe Ala Gly Asp Met Arg
   180 185 190
   Leu Phe Val Lys Asp Asn Asp Gln Lys Lys Ala Glu Ile Lys Ile Arg
   195 200 205
   Met Pro Glu Lys Ile Lys Glu Thr Lys Ser Glu Tyr Pro Tyr Val Ser
   210 215 220
   Ser Tyr Gly Asn Val Ile Glu Leu Gly Glu Gly Asp Leu Ser Lys Asn
   225 230 235 240
   Lys Pro Asp Asn Leu Thr Lys Met Glu Ser Gly Lys Ile Tyr Ser Asp
   245 250 255
   Ser Glu Lys Gln Gln Tyr Leu Leu Lys Asp Asn Ile Ile Leu Arg Lys
   260 265 270
   Gly Tyr Ala Leu Lys Val Thr Thr Tyr Asn Pro Gly Lys Thr Asp Met
   275 280 285
   Leu Glu Gly Asn Gly Val Tyr Ser Lys Glu Asp Ile Ala Lys Ile Gln
   290 295 300
   Lys Ala Asn Pro Asn Leu Arg Ala Leu Ser Glu Thr Thr Ile Tyr Ala
   305 310 315 320
   Asp Ser Arg Asn Val Glu Asp Gly Arg Ser Thr Gln Ser Val Leu Met
   325 330 335
   Ser Ala Leu Asp Gly Phe Asn Ile Ile Arg Tyr Gln Val Phe Thr Phe
   340 345 350
   Lys Met Asn Asp Lys Gly Glu Ala Ile Asp Lys Asp Gly Asn Leu Val
   355 360 365
   Thr Asp Ser Ser Lys Leu Val Leu Phe Gly Lys Asp Asp Lys Glu Tyr
   370 375 380
   Thr Gly Glu Asp Lys Phe Asn Val Glu Ala Ile Lys Glu Asp Gly Ser
   385 390 395 400
   Met Leu Phe Ile Asp Thr Lys Pro Val Asn Leu Ser Met Asp Lys Asn
   405 410 415
   Tyr Phe Asn Pro Ser Lys Ser Asn Lys Ile Tyr Val Arg Asn Pro Glu
   420 425 430
   Phe Tyr Leu Arg Gly Lys Ile Ser Asp Lys Gly Gly Phe Asn Trp Glu
   435 440 445
   Leu Arg Val Asn Glu Ser Val Val Asp Asn Tyr Leu Ile Tyr Gly Asp
   450 455 460
   Leu His Ile Asp Asn Thr Arg Asp Phe Asn Ile Lys Leu Asn Val Lys
   465 470 475 480
   Asp Gly Asp Ile Met Asp Trp Gly Met Lys Asp Tyr Lys Ala Asn Gly
   485 490 495
   Phe Pro Asp Lys Val Thr Asp Met Asp Gly Asn Val Tyr Leu Gln Thr
   500 505 510
   Gly Tyr Ser Asp Leu Asn Ala Lys Ala Val Gly Val His Tyr Gln Phe
   515 520 525
   Leu Tyr Asp Asn Val Lys Pro Glu Val Asn Ile Asp Pro Lys Gly Asn
   530 535 540
   Thr Ser Ile Glu Tyr Ala Asp Gly Lys Ser Val Val Phe Asn Ile Asn
   545 550 555 560
   Asp Lys Arg Asn Asn Gly Phe Asp Gly Glu Ile Gln Glu Gln His Ile
   565 570 575
   Tyr Ile Asn Gly Lys Glu Tyr Thr Ser Phe Asn Asp Ile Lys Gln Ile
   580 585 590
   Ile Asp Lys Thr Leu Asn Ile Lys Ile Val Val Lys Asp Phe Ala Arg
   595 600 605
   Asn Thr Thr Val Lys Glu Phe Ile Leu Asn Lys Asp Thr Gly Glu Val
   610 615 620
   Ser Glu Leu Lys Pro His Arg Val Thr Val Thr Ile Gln Asn Gly Lys
   625 630 635 640
   Glu Met Ser Ser Thr Ile Val Ser Glu Glu Asp Phe Ile Leu Pro Val
   645 650 655
   Tyr Lys Gly Glu Leu Glu Lys Gly Tyr Gln Phe Asp Gly Trp Glu Ile
   660 665 670
   Ser Gly Phe Glu Gly Lys Lys Asp Ala Gly Tyr Val Ile Asn Leu Ser
   675 680 685
   Lys Asp Thr Phe Ile Lys Pro Val Phe Lys Lys Ile Glu Glu Lys Lys
   690 695 700
   Glu Glu Glu Asn Lys Pro Thr Phe Asp Val Ser Lys Lys Lys Asp Asn
   705                 710                 715                 720
   Pro Gln Val Asn His Ser Gln Leu Asn Glu Ser His Arg Lys Glu Asp
                   725                 730                 735
   Leu Gln Arg Glu Glu His Ser Gln Lys Ser Asp Ser Thr Lys Asp Val
               740                 745                 750
   Thr Ala Thr Val Leu Asp Lys Asn Asn Ile Ser Ser Lys Ser Thr Thr
           755                 760                 765
   Asn Asn Pro Asn Lys
       770

## Description

### BACKGROUND OF THE INVENTION

*Streptococcus pneumoniae* is a gram positive bacterium which is a major causative agent in invasive infections in animals and humans, such as sepsis, meningitis, otitis media and lobar pneumonia (Tuomanen, et al. NEJM 322:1280-1284 (1995)). As part of the infective process, pneumococci readily bind to non-inflamed human epithelial cells of the upper and lower respiratory tract by binding to eukaryotic carbohydrates in a lectin-like manner (Cundell et al., Micro. Path. 17:361-374 (1994)). Conversion to invasive pneumococcal infections for bound bacteria may involve the local generation of inflammatory factors which may activate the epithelial cells to change the number and type of receptors on their surface (Cundell, et al., Nature, 377:435-438 (1995)). Apparently, one such receptor, platelet activating factor (PAF) is engaged by the pneumococcal bacteria and within a very short period of time (minutes) from the appearance of PAF, pneumococci exhibit strongly enhanced adherence and invasion of tissue. Certain soluble receptor analogs have been shown to prevent the progression of pneumococcal infections (Idanpaan-Heikkila et al., J. Inf. Dis., 176:704-712 (1997)). A number of various other proteins have been suggested as being involved in the pathogenicity of *S. pneumoniae* but only some have been confirmed as virulence factors. Despite the fact that there are capsule conjugates currently in trial, there still remains a need for identifying additional polypeptides having epitopes in common from various strains of *S. pneumoniae* in order to utilize such polypeptides as vaccines to provide protection against a wide variety of *S. pneumoniae* serotypes.

WO 98/18930 discloses Streptococcus pneumoniae antigens which are potential candidates for the preparation of vaccine.

### BRIEF SUMMARY OF THE INVENTION

The invention disclosed herein relates to a vaccine comprising as active ingredient a polypeptide of the pneumococcal organism *Streptococcus pneumoniae*, comprising the seq ID no 6.

More specifically, the present invention discloses Sp128 (SEQ ID NO:6), composed of 664 amino acid residues. Sp128 has been found to confer protective properties on animals immunized with said polypeptide**.**

The present invention also relates to the field of bacterial antigens and their use, for example, as immunogenic agents in humans and animals to stimulate an immune response. More specifically, it relates to the vaccination of mammalian species with one or more recombinant polypeptides produced according to the invention disclosed herein, such recombinant polypeptides being derived from *Streptococcus pneumoniae.*

In accordance with the present invention, such proteins serve as a mechanism for stimulating production of antibodies that protect the vaccine recipient against infection by a wide range of capsular serotypes of pathogenic *S.* pneumoniae.

In a particular aspect, the present invention relates to the prevention and treatment of pneumococcal infections such as infections of the middle ear, nasopharynx, lung and bronchial areas, blood, CSF, and the like, that are caused by pneumococcal bacteria.

The present invention further relates to vaccines prepared from the novel polypeptide, disclosed herein. In addition, examples of the use of such polypeptide as a vaccine for the protection of mammals are likewise disclosed.

### BRIEF DESCRIPTION OF THE DRAWINGS

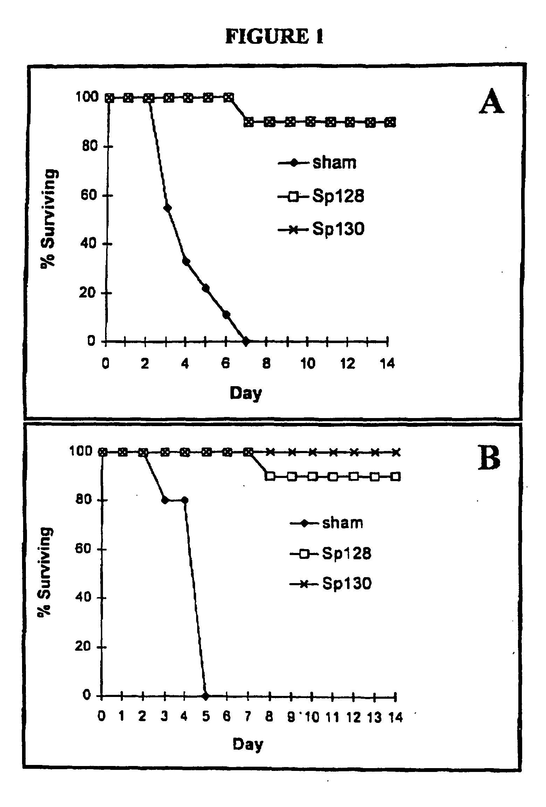

Figure 1 shows the results of 2 experiments (Figs 1A and 1B, respectively), using the same preparations of Sp128 and Sp130 (for background purposes) polypeptides. The results demonstrate that active immunization with recombinant Sp128 or Sp130 polypeptides derived from the pneumococcal strain Norway serotype 4 is able to protect mice from death in a model of pneumococcal sepsis using the heterologous strain SJ2 (serotype 6B). In these 2 experiments, 90% and 100%, respectively, of the mice immunized with Sp130 survived the 14 day observation period following challenge with about 400 CFU (colony forming units) of pneumococci. Conversely, 100% of sham immunized mice (injected only with PBS (phosphate-buffered saline) plus adjuvant) died during the same period. In addition, for both experiments, 90% of the mice immunized with Sp128 survived the same 14 day observation period.

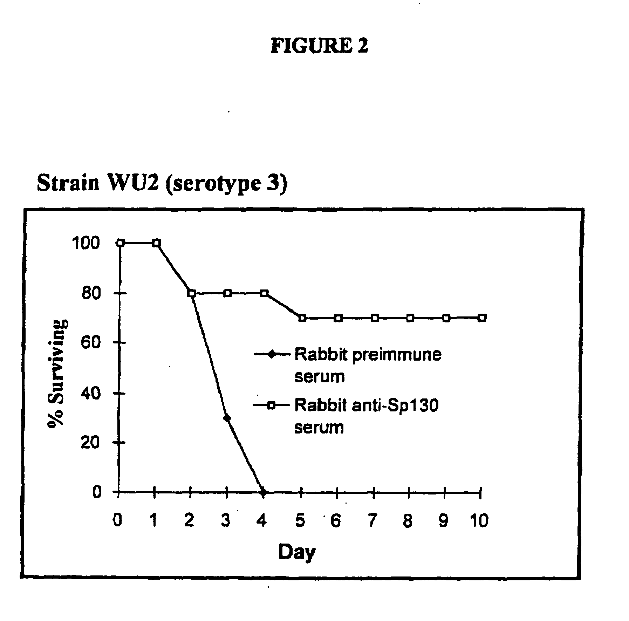

Figure 2 shows the results of passive administration of rabbit antiserum raised against Sp130 derived from Norway serotype 4. Such administration was able to protect mice in the pneumococcal sepsis model using a heterologous strain. More specifically, 70% of the mice immunized with the Sp130 antiserum survived the 10 day observation period after challenge with 1400 CFU of strain WU2 (serotype 3). In addition, 100% of the mice immunized with a control serum (collected before immunization) died by day 4.

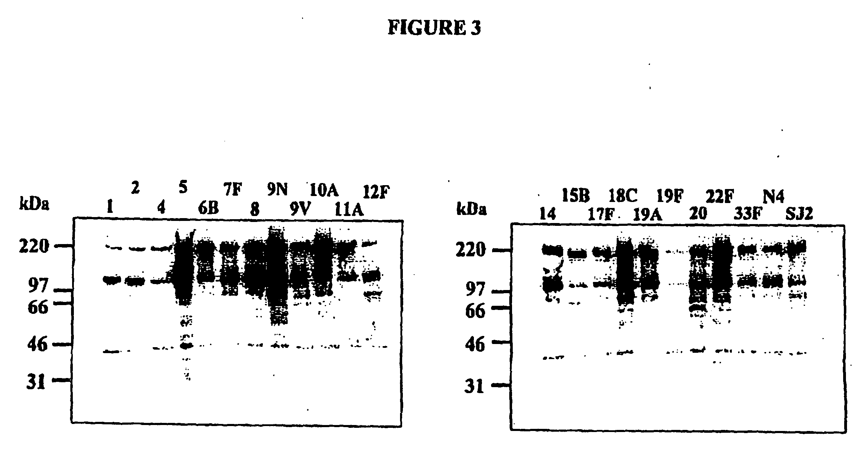

Figure 3 is a western blot showing reactivity of antisera raised against recombinant Sp130 (derived from strain Norway serotype 4) with whole cell lysates of heterologous strains. All *S. pneumoniae* strains tested showed a band of molecular weight about 220 kD, the expected mass for a protein containing both the Sp128 and Sp130 sequences, indicating that this protein was present in all of the tested strains. Tested strains included isolates from each of the pneumococcal serotypes represented in the currently used 23-valent polysaccharide vaccine.

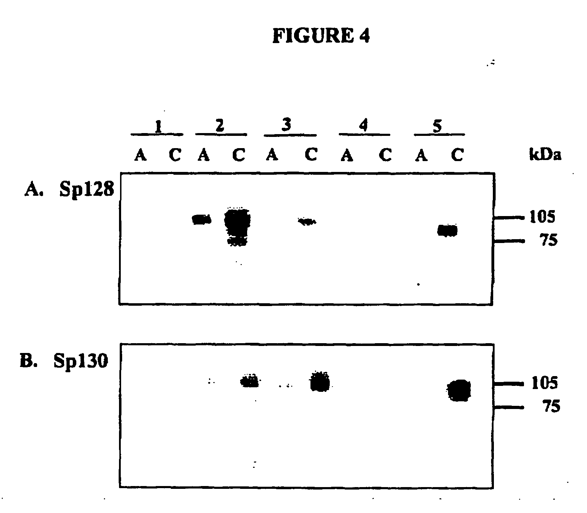

Figure 4 is a western blot showing the reactivity of patient sera with either Sp128 or Sp130. Fig. 4A shows the results for Sp128. Fig. 4B shows the results for Sp130. The recombinant proteins were resolved by SDS-PAGE and transferred to nitrocellulose. Sera were collected from 5 patients (indicated by number at the top) at two different times. First collection (denoted "A" for "acute serum") was soon after onset of illness; second collection (denoted "C" for "convalescent") was made 8 to 30 days later. These sera were used to probe the blots. The results show that for patients 2, 3 and 5, convalescent serum reacted more strongly with Sp128 and Sp130 than did the corresponding acute serum. Such findings constitute indirect evidence that both Sp128 and Sp130 are expressed by *S. pneumoniae* during this phase of infection.

### DETAILED SUMMARY OF THE INVENTION

In accordance with the present invention there is disclosed herein a recombinant polypeptide corresponding to Sp128 (SEQ ID NO: 6).

It is an object of the present invention to provide methods of utilizing these recombinant polypeptide, as a means of immunizing animals, especially mammals, most especially humans, against a variety of microbial infections, especially pneumococcal infections.

It is a further object of the present invention to provide a polypeptide, as disclosed herein, whether derived from natural sources or prepared by means of recombinant technology, for use in immunizing animals, especially mammals, most especially humans, against pneumococcal infection.

It is a still further object of the present invention to provide vaccines that include the polypeptide obtained from *S. pneumoniae,* including the polypeptide prepared by recombinant means (i.e., recombinant polypeptides and proteins).

The polypeptide, of the vaccines disclosed as expression products according to the invention may be in "enriched form." As used herein, the term " enriched" means that the concentration of the material is at least about 2, 5, 10, 100, or 1000 times its natural concentration (for example), advantageously 0.01%, by weight, preferably at least about 0.1% by weight. Enriched preparations of about 0.5%, 1%, 5%, 10%, and 20% by weight are also contemplated.

"Isolated" in the context of the present invention with respect to polypeptides means that the material is removed from its original environment (e.g., the natural environment if it is naturally occurring). For example, a naturally-occurring polynucleotide or polypeptide present in a living organism is not isolated, but the same polypeptide, separated from some or all of the co-existing materials in the natural system, is isolated. Such polypeptides could be part of a composition, and still be isolated in that such composition is not part of its natural environment. The polypeptides of the vaccines disclosed herein are preferably provided in an isolated form, and preferably are purified to homogeneity.

The recombinant or immunogenic polypeptides disclosed in accordance with the present invention may also be in " purified" form. The term " purified" does not require absolute purity; rather, it is intended as a relative definition, and can include preparations that are highly purified or preparations that are only partially purified, as those terms are understood by those of skill in the relevant art. For example, individual clones isolated from a cDNA library have been conventionally purified to electrophoretic homogeneity. Purification of starting material or natural material to at least one order of magnitude, preferably two or three orders, and more preferably four or five orders of magnitude is expressly contemplated. Furthermore, claimed polypeptide which has a purity of preferably 0.001%, or at least 0.01% or 0.1%; and even desirably 1% by weight or greater is expressly contemplated.

At the simplest level, the amino acid sequence corresponding to all or part of the polypeptide according to the present invention can be synthesized using commercially available peptide synthesizers.

The polypeptide vaccines of the present invention may be recombinant polypeptides, natural polypeptides or synthetic polypeptides, preferably recombinant polypeptides.

"Recombinant," as used herein, means that a protein is derived from recombinant (e.g., microbial or mammalian) expression systems. "Microbial" refers to recombinant proteins made in bacterial or fungal (e.g., yeast) expression systems. As a product, "recombinant microbial" defines a protein essentially free of native endogenous substances and unaccompanied by associated native glycosylation. Protein expressed in most bacterial cultures, e.g., E. coli, will be free of glycosylation modifications that might normally accur in yeast or mammalian expression systems. Thus, the patterns of such post-translational modifications will differ with the expression system. However, all such variants are considered to lie within the disclosure of the present invention.

Described is also an isolated antibody that binds specifically to a polypeptide of the invention. Such an antibody may be a monoclonal antibody, possibly produced by a hybridoma cell line, and may also include a recombinantly produced antibody formed by introducing into a suitable cell line the gene sequences required for producing an antibody specific for the polypeptide vaccines disclosed herein.

The present invention is also directed to a vaccine comprising a *S. pneumoniae* polypeptide, disclosed herein, suspended in a pharmaceutically acceptable diluent, carrier or excipient, provided that said polypeptide is present in an amount effective to elicit protective antibodies in an animal against an organism related to the genus *Streptococcus,* preferably an organism of the genus *Streptococcus,* and most preferably where the organism is *Streptococcus pneumoniae.*

A vaccine disclosed according to the present invention may also include a vaccine comprising a microbial organism transformed with polynucleotides, and thereby expressing the polypeptide, of Sp128 (SEQ ID NOS: 6). The transformed microorganism is selected from the group consisting of Salmonella, Mycobacteria, Streptococcus, poxviruses, and adenoviruses.

Fragments or portions of the polypeptides of the present invention may be employed for producing the corresponding full-length polypeptide by peptide synthesis; therefore, the fragments may be employed as intermediates for producing the full-length polypeptides.

As noted, the polypeptides, or cells expressing them, can be used as an immunogen to produce antibodies thereto. These antibodies can be, for example, polyclonal, monoclonal, chimeric, single chain, Fab fragments, or the product of an Fab expression library. Various procedures known in the art may be used for the production of polyclonal antibodies, especially where these are in the form of antisera raised against the polypeptides, or fragments thereof, according to the present invention. Such antisera find use in immunization against pneumococcal infection.

Antibodies generated against a polypeptide vaccine corresponding to a sequence of the present invention can be obtained by direct injection of the polypeptide into an animal or by administering the polypeptide to an animal, preferably a nonhuman. The antibody so obtained will then bind the polypeptide itself. In this manner, even a sequence encoding only a fragment of the polypeptide can be used to generate antibodies binding the whole native polypeptide.

For preparation of monoclonal antibodies, any technique which provides antibodies produced by continuous cell line cultures can be used. Examples include the hybridoma technique (Kohler and Milstein, 1975, Nature, 256:495-497), the trioma technique, the human B-cell hybridoma technique (Kozbor et al., 1983, Immunology Today 4:72), and the EBV-hybridoma technique to produce human monoclonal antibodies (Cole, et al., 1985, in Monoclonal Antibodies and Cancer Therapy, Alan R. Liss, Inc., pp. 77-96).

Thus, the present invention also relates to the use of the novel polypeptides disclosed herein, for the production of lymphocytes, or hybridoma cells, producing monoclonal antibodies against such polypeptides, for preventing or attenuating an infection caused by a member of the genus streptococcus in an animal.

Techniques described for the production of single chain antibodies (U.S. Patent 4,946,778) can be adapted to produce single chain antibodies to immunogenic polypeptide products of this invention.

A vaccine in accordance with the present invention includes the polypeptide hereinabove described. When employing more than one polypeptide, such as two or more polypeptides may be used as a physical mixture or as a fusion of two or more polypeptides. The fusion fragment or fusion polypeptide may be produced, for example, by recombinant techniques or by the use of appropriate linkers for fusing previously prepared polypeptides or active fragments.

In another aspect, the invention relates to passive immunity vaccines formulated from antibodies against a polypeptide of the present invention. Such passive immunity vaccines can be utilized to prevent and/or treat streptococcal infections in patients. In this manner, according to a further aspect of the invention, a vaccine can be produced from a synthetic or recombinant polypeptide of the present invention or an antibody against such polypeptide.

As already described, another aspect the present invention relates to one or more antibodies (monoclonal, polyclonal or sera) to the polypeptide of the invention as described above for the prophylaxis and/or treatment of diseases that are caused by streptococcal bacteria. In particular, the invention relates to the prophylaxis and/or treatment of infectious diseases that are caused by *S. pneumoniae.* In a still further preferred aspect, the invention relates to the prophylaxis and/or treatment of otitis media, nasopharyngeal and bronchial infections, and the like in humans by utilizing a vaccine of the present invention.

Generally, vaccines are prepared as injectables, in the form of aqueous solutions or suspensions. Vaccines in an oil base are also well known such as for inhaling. Solid forms which are dissolved or suspended prior to use may also be formulated. Pharmaceutical carriers, diluents and excipients are generally added that are compatible with the active ingredients and acceptable for pharmaceutical use. Examples of such carriers include, but are not limited to, water, saline solutions, dextrose, or glycerol. Combinations of carriers may also be used.

Vaccine compositions may further incorporate additional substances to stabilize pH, or to function as adjuvants, wetting agents, or emulsifying agents, which can serve to improve the effectiveness of the vaccine.

Vaccines are generally formulated for parenteral administration and are injected either subcutaneously or intramuscularly. Such vaccines can also be formulated as suppositories or for oral administration, using methods known in the art, or for administration through nasal or respiratory routes.

The amount of vaccine sufficient to confer immunity to pathogenic bacteria is determined by methods well known to those skilled in the art. This quantity will be determined based upon the characteristics of the vaccine recipient and the level of immunity required. Typically, the amount of vaccine to be administered will be determined based upon the judgment of a skilled physician. Where vaccines are administered by subcutaneous or intramuscular injection, a range of .5 to 500 µg purified protein may be given.

The present invention is also directed to a vaccine in which a polypeptide of the present invention is delivered or administered in the form of a polynucleotide encoding the polypeptide or active fragment, whereby the polypeptide or active fragment is produced *in vivo.* The polynucleotide may be included in a suitable expression vector and combined with a pharmaceutically acceptable carrier.

In addition, the polypeptides of the present invention can be used as immunogens to stimulate the production of antibodies for use in passive immunotherapy.

The polypeptides of the invention can be synthetically produced by conventional peptide synthesizers.

Mature proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook, et al., Molecular Cloning: A Laboratory Manual, Second Edition, Cold Spring Harbor, N.Y., (1989).

Transcription of the DNA encoding the polypeptides of the present invention by higher eukaryotes is increased by inserting an enhancer sequence into the vector. Enhancers are cis-acting elements of DNA, usually about from 10 to 300 bp that act on a promoter to increase its transcription. Examples including the SV40 enhancer on the late side of the replication origin bp 100 to 270, a cytomegalovirus early promoter enhancer, the polyoma enhancer on the late side of the replication origin, and adenovirus enhancers.

Generally, recombinant expression vectors will include origins of replication and selectable markers permitting transformation of the host cell, e.g., the ampicillin resistance gene of E. coli and S. cerevisiae TRP1 gene, and a promoter derived from a highly-expressed gene to direct transcription of a downstream structural sequence. Such promoters can be derived from operons encoding glycolytic enzymes such as 3-phosphoglycerate kinase (PGK), α-factor, acid phosphatase; or heat shock proteins, among others. The heterologous structural sequence is assembled in appropriate phase with translation initiation and termination sequences. Optionally, the heterologous sequence can encode a fusion protein including an N-terminal identification peptide imparting desired characteristics, e.g., stabilization or simplified purification of expressed recombinant product.

Useful expression vectors for bacterial use are constructed by inserting a structural DNA sequence encoding a desired protein- together with suitable translation initiation and termination signals in operable reading phase with a functional promoter. The vector will comprise one or more phenotypic selectable markers and an origin of replication to ensure maintenance of the vector and to, if desirable, provide amplification within the host. Suitable prokaryotic hosts for transformation include E. coli, Bacillus subtilis, Salmonella typhimurium and various species within the genera Pseudomonas, Streptomyces, and Staphylococcus, although others may also be employed as a matter of choice, including streptococcal species, especially *S. pneumoniae.*

In accordance with the invention, microbial organisms genetically transformed with polynucleotides expressing Sp128, may themselves be used as living vaccine delivery vehicles. Examples include, but are in no way limited to, *Salmonella* species, *Mycobacterium* species, *Streptococcus* species, poxviruses, adenoviruses, and the like. In addition, transgenic edible plants may also be candidates for vaccine delivery.

As a representative but nonlimiting example, useful expression vectors for bacterial use can comprise a selectable marker and bacterial origin of replication derived from commercially available plasmids comprising genetic elements of the well known cloning vector pBR322 (ATCC 37017). Such commercial vectors include, for example, pKK223-3 (Amersham Pharmacia Biotech, Piscataway, NJ, USA) and pGEM1 (Promega, Madison, WI, USA). These pBR322 "backbone" sections are combined with an appropriate promoter and the structural sequence to be expressed.

Following transformation of a suitable host strain and growth of the host strain to an appropriate cell density, the selected promoter is induced by appropriate means (e.g., temperature shift or chemical induction) and cells are cultured for an additional period.

Cells are typically harvested by centrifugation, disrupted by physical or chemical means, and the resulting crude extract retained for further purification.

Microbial cells employed in expression of proteins can be disrupted by any convenient method, including freeze-thaw cycling, sonication, a french press, mechanical disruption, or use of cell lysing agents, such methods are well know to those skilled in the art. However, preferred are host cells which secrete the polypeptide of the invention and permit recovery of the polypeptide from the culture media.

Various mammalian cell culture systems can also be employed to express recombinant protein. Examples of mammalian expression systems include the COS-7 lines of monkey kidney fibroblasts, described by Gluzman, Cell, 23:175 (1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 splice, and polyadenylation sites may be used to provide the required nontranscribed genetic elements.

The polypeptides can be recovered and/or purified from recombinant cell cultures by well-known protein recovery and purification methods. Such methodology may include ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Protein refolding steps can be used, as necessary, in completing configuration of the mature protein. In this respect, chaperones may be used in such a refolding procedure. Finally, high performance liquid chromatography (HPLC) can be employed for final purification steps.

The polypeptides that are useful as immunogens in the present invention may be a naturally purified product, or a product of chemical synthetic procedures, or produced by recombinant techniques from a prokaryotic or eukaryotic host (for example, by bacterial, yeast, higher plant, insect and mammalian cells in culture). Depending upon the host employed in a recombinant production procedure, the polypeptides of the present invention may be glycosylated or may be non-glycosylated.

Procedures for the isolation of the individually expressed polypeptides may be isolated by recombinant expression/isolation methods that are well-known in the art. Typical examples for such isolation may utilize an antibody to a conserved area of the protein or to a His tag or cleavable leader or tail that is expressed as part of the protein structure.

Specific embodiments of the invention will now be further described in more detail in the following nonlimiting examples.

### EXAMPLE 1

### Active Protection with Anti-Sp128 and Anti-Sp130 (for reference purposes)

### A. Cloning, Expression, and Purification of Sp128 and Sp130.

The genomic DNA used as target for amplification using the polymerase chain reaction was isolated from *Streptococcus pneumoniae* (Norway strain - serotype 4), the same strain used for genomic sequencing. The nucleotide sequence of the gene fragments encoding Sp128 (SEQ ID NO: 5) and Sp130 (SEQ ID NO: 7) with the corresponding amino acid sequence for polypeptides Sp128 (SEQ ID NO: 6) and Sp130 (SEQ ID NO: 8) are provided in the Sequence Listing.

Primers (SEQ ID NOS: 1 - 4) were designed so as to amplify either Sp128 or Sp130 gene fragments and allow their cloning into the *E. coli* expresssion vector pQE10 with, for example, subsequent expression of a histidine-tagged protein product for purification by Nickel-affinity chromatography.

Thus, cloning of the fragments amplified by the primers of SEQ ID NOS: 1 and 2 results in the polypeptide of SEQ ID NO: 6 (denoted Sp128), while cloning of the fragment amplified using the primers disclosed in SEQ ID NOS: 3 and 4 result in the polypeptide of SEQ ID NO: 8 (denoted Sp130).

### B. Vaccination With Sp128 and Sp130 Results in Protection Against Lethal S. pneumoniae Challenge

In each of the experiments shown in Figure 1, C3H/HeJ mice (10 per group) were immunized intraperitoneally (i.p.) with either Sp128 or Sp130 protein (15 µg in 50 µl PBS (phosphate buffered saline) emulsified in 50 µl complete Freund's adjuvant (CFA)). A group of 10 mice were sham-immunized with PBS and CFA only.

A second immunization of 15 µg protein with incomplete Freund's adjuvant (IFA) was administered 3 weeks later (with the sham-immunized group receiving PBS and IFA).

Blood was drawn (retro-orbital bleed) at week 7. Sera from each group were pooled for analysis of anti-Sp128 and anti-Sp130 antibody by ELISA. Mice were challenged at week 8 by intraperitoneal injection of approximately 400 CFU (colony forming units) of *S. pneumoniae* strain SJ2 (capsular serotype 6B). In preliminary experiments, the median infection dose (LD₅₀) of this strain was determined to be approximately 10 CFU. Mice were monitored for 14 days of survival.

Both experiments shown in Figure 1 used the same preparations of recombinant Sp128 and Sp130.

In the experiment shown in Figure 1A, 7-week serum collected from the 10 mice immunized with either Sp128 or Sp130 each had an endpoint ELISA titer of 1:2,048,000 and 1:1,024,000, respectively. No anti-Sp128 or anti-Sp130 antibody was detected in sera from sham-imunized mice. Ninety percent of the mice immunized with either Sp128 or Sp130 protein survived the challenge (406 CFU of pneumococci) for the extent of the study (14 days). One hundred percent of sham-immunized mice were dead by day 7.

In the experiment shown in Figure 1B, 7-week sera collected from the 10 mice immunized with either Sp128 or Sp130 each had an endpoint ELISA titer of 1:1,024,000 and 1:512,000, respectively. No anti-Sp128 or anti-Sp130 antibody was detected in sera from sham-imunized mice. Ninety and one hundred percent of the mice immunized with either Sp128 or Sp130 protein, respectively, survived the challenge (404 CFU of pneumococci) for the extent of the study (14 days). One hundred percent of sham-immunized mice were dead by day 5.

These data indicate that immunization of mice with either recombinant Sp128 or Sp130 proteins elicit a response capable of protecting against systemic pneumococcal infection and subsequent death. Cross protection is demonstrated by the fact that the recombinant pneumococcal protein was generated based on capsular serotype 4 DNA sequence, while the challenge was with the heterologous strain SJ2 (capsular serotype 6B).

### EXAMPLE 2

### Passive Protection With Anti-Sp130 Antisera

### A. Generation of Rabbit Immune Sera

Following collection of pre-immune serum, a New Zealand White rabbit was immunized with 250 µg of Sp130 (SEQ ID NO:8) in complete Freund's adjuvant. The rabbit was given 2 boosts of 125 µg Sp130 in incomplete Freund's adjuvant on days 21 and 52, and bled on days 31 and 62.

### B. Passive Protection in Mice

BALB/cByJ mice (10 per group) were passively immunized by 2 i.p. injections of 100 µl of rabbit serum. The first injection was administered 24 hours before challenge with 1400 CFU of *S. pneumoniae* strain WU2, and the second injection was given 4 hours after challenge. Figure 2 shows the survival of mice after infection with WU2 (capsular serotype 3) strain. In preliminary experiments, the LD₅₀ of this strain was determined to be approximately 100 CFU.

Figure 2 shows the survival of mice injected with 1400 CFU of strain WU2. As shown therein, 70% of the mice immunized with rabbit immune serum raised against Sp130 protein survived the 10 day observation period. Of the mice immunized with the control serum (collected from a rabbit prior to immunization), 100% died by day 4.

These data suggest that the protection against pneumococcal infection resulting from immunization with Sp130 is antibody-mediated, since the mice were protected by passive transfer of serum from a hyperimmunized rabbit. As seen in the previously described mouse active challenge experiments, serum directed against recombinant Sp130 protein cloned from a serotype 4 strain was protective against challenge with a heterologous strain, WU2 (capsular serotype 3).

### EXAMPLE 3

### Conservation of Sp128-Sp130 Among Strains of S. pneumoniae

### A. Western Blotting

The pneumococcal strains used in this experiment were obtained from the American Type Culture Collection (Rockville, MD) and include one isolate from each of the serotypes in the currently used multivalent pneumococcal vaccine.

For total cell lysates, pneumocci were grown to mid-logarithmic phase (absorbance at 620 nm was 0.4 to 0.6) in 2 ml Todd-Hewitt broth with 5% yeast extract (from Difco, Detroit, MI) at 37°C. Bacteria were harvested by centrifugation and washed twice with water. Pellets (consisting of sedimented cells) were resuspended in 200 µl of lysis buffer (0.01% sodium dodecyl sulfate, 0.15 M sodium citrate, and 0.1% sodium deoxycholate) and incubated at 37°C for 30 min, then diluted in an equal volume of 2X SSC (0.3 M NaCl, 0.03 M sodium citrate). Polypeptides in the lysates were resolved on SDS-PAGE (sodium dodecyl sulfate polyacrylamide gel electrophoresis), transferred to nitrocellulose membranes (Bio-Rad Laboratories, Hercules, CA) and probed with antibody by conventional Western Blotting procedures. Sera from a New Zealand White rabbit immunized with Sp130 (as per Example 2, supra) was used at a dilution of 1:3000. Bound antibody was detected with peroxidase-conjugated sheep anti-rabbit IgG using a chemiluminescence kit from Amersham Inc. (Cambridge, MA).

The rabbit anti-Sp130 sera revealed 2 major bands with apparent molecular weights of 110 kD and 220 kD in all 23 pneumococcal lysates tested (as shown in Figure 3) .

These data show that Sp130 is expressed and shares common antigenic epitopes among strains of the 23 pneumococcal capsular serotypes represented in the currently used polysaccharide vaccine.

### EXAMPLE 4

### Immunogenicity of Sp128 and Sp130 in Humans

Sera from patients with culture-proven pneumococcal bacteremia were used in Western blots containing recombinant Sp128 or Sp130 protein. In the experiment shown in Figure 4, sera from 5 patients (indicated by numerals 1 through 5) were diluted 1:3000 and used to probe blots containing Sp128 (SEQ ID NO:6) or Sp130 (SEQ ID N0:8).

The lanes labeled "A" (for "acute") were probed with serum collected shortly after diagnosis of pneumococcal infection; lanes denoted "C" (for "convalescent") were probed with serum collected either 1 month later (patients 1, 2, and 3) or 8 days after the first serum collection (patients 4 and 5). For patients 2, 3, and 5, reactivity of the convalescent serum with Sp128 and Sp130 was stronger than that of the corresponding acute serum.

Other experiments (not depicted in the figure) showed that convalescent sera from 17 patients with pneumococcal infections were tested individually for reactivity with either Sp128 or Sp130. Thus, 10 and 15 of the 23 sera were found to bind (on a Western Blot) Sp128 and Sp130, respectively.

These experiments indicate that Sp128 and Sp130 (the latter to a greater extent), are recognized by the human immune system and suggest that antibodies able to bind the Sp128-Sp130 protein may be produced during natural *S. pneumoniae* infection in humans. Further, this provides indirect evidence that Sp128 and Sp130 are expressed *in vivo* by *S. pneumoniae* during this phase of infection, and thus may be available as targets for immunoprophylaxis, immunotherapy, or to provide anamnestic immune responses in subjects vaccinated with these proteins. Since the patients were infected with a variety of pneumococcal strains, these data also support the idea that Sp130 is more antigenically conserved than Sp128.
Sentence 1. A vaccine comprising a polypeptide, including immunogenic fragments thereof, having an amino acid sequence at least 65% identical to the amino acid sequence of SEQ ID NO:6.
Sentence 2. The vaccine of sentence 1 wherein said amino acid sequence is at least 80% identical to the amino acid sequence of SEQ ID NO:6.
Sentence 3. The vaccine sentence 1 wherein said amino acid sequence is at least 95% identical to the amino acid sequence of SEQ ID NO:6.
Sentence 4. The vaccine of sentence 1 wherein said amino acid sequence is identical to the amino acid sequence of SEQ ID N0:6.
Sentence 5. A vaccine comprising a polypeptide, including immunogenic fragments thereof, having an amino acid sequence at least 65% identical to the amino acid sequence of SEQ ID NO:8.
Sentence 6. The vaccine of sentence 5 wherein said amino acid sequence is at least 80% identical to the amino acid sequence of SEQ ID NO:8.
Sentence 7. The vaccine of sentence 5 wherein said amino acid sequence is at least 95% identical to the amino acid sequence of SEQ ID NO:8.
Sentence 8. The vaccine of sentence 5 wherein said amino acid sequence is identical to the amino acid sequence of SEQ ID N0:8.
Sentence 9. An antiserum produced by immunizing an animal with a polypeptide selected from the group consisting of the polypeptides of sentences 1, 2, 3, 4, 5, 6, 7, and 8.
Sentence 10. An isolated antibody that binds specifically to a polypeptide selected from the group consisting of the polypeptides according to sentences 1, 2, 3, 4, 5, 6, 7, and 8.
Sentence 11. The antibody of sentence 10 wherein the antibody is a monoclonal antibody.
Sentence 12. An engineered cell producing a monoclonal antibody of sentence 11.
Sentence 13. An antiserum produced by immunizing an animal with the polypeptide of SEQ ID NO: 6.
Sentence 14. An antiserum produced by immunizing an animal with the polypeptide of SEQ-ID NO: 8.
Sentence 15. An isolated recombinant antibody that binds specifically to a polypeptide selected from the group consisting of the polypeptides of sentences 1,2,3,4,5,6,7, and 8.
Sentence 16. A vaccine comprising:
   a. one or more *S. pneumoniae* polypeptides selected from the group consisting of the polypeptides of sentences 1, 2, 3, 4, 5, 6, 7, and 8; and
   b. a pharmaceutically acceptable diluent , carrier or excipient;
   wherein said polypeptide is present in an amount effective to elicit protective antibodies in an animal against an organism of the genus *Streptococcus.*
Sentence 17. A method of preventing or attenuating an infection caused by a member of the genus *Streptococcus* in an animal, comprising administering to said animal a polypeptide selected from the group consisting of the polypeptides of sentences 1, 2, 3, 4, 5, 6, 7, and 8, and wherein said polypeptide is administered in an amount effective to prevent or attenuate said infection.
Sentence 18. A method of preventing pneumococcal infection by administering to an animal the vaccine according to claim 16.
Sentence 19. A method of preventing or attenuating an infection caused by a member of the genus *Streptococcus* in an animal, comprisin administering to said animal an antibody according sentence 10, wherein said antibody is administered in an amount effective to prevent or attenuate said infection.
Sentence 20. A vaccine comprising a microbial organism transformed with polynucleotides, and thereby expressing the polypeptides, or fragments thereof, selected from the group consisting of Sp128 and Sp130.
Sentence 21. A method of preventing or attenuating an infection caused by a member of the genus *Streptococcus* in an animal, comprising administering to said animal a vaccine according to sentence 20, wherein said antibody is administered in an amount effective to prevent or attenuate said infection.
Sentence 22. The vaccine according to Sentence 20, wherein said transformed microorganism is selected from the group consisting of Salmonella, Mycobacteria, Streptococcus, poxviruses, and adenoviruses.

## Claims

1. A vaccine comprising as an active ingredient a polypeptide, comprising the sequence of SEQ ID NO: 6.

2. A vaccine composition, according to claim 1 together with a pharmaceutically acceptable carrier; wherein the polypeptide is present in an amount effective to elicit antibodies in an animal against an organism of the genus *Streptococcus.*

3. A vaccine according to claim 1 wherein the polypeptide is provided by a microbial organism transformed with polynucleotides, and thereby expressing the polypeptide of SEQ ID NO: 6, as an active ingredient of said vaccine.

4. The vaccine according to claim 3 for use in preventing or attenuating an infection caused by a member of the genus *Streptococcus* in an animal

5. The vaccine according to claim 3, wherein said transformed microorganism is selected from the group consisting of Salmonella, Mycobacteria, Streptococcus, poxviruses, and adenoviruses.

6. Use of a polypeptide comprising the sequence of SEQ ID NO: 6 for the manufacture of a pharmaceutical preparation for preventing or attenuating an infection caused by a member of the genus *Streptococcus* in an animal.

7. Use of an antibody that binds specifically to a polypeptide comprising the sequence of SEQ ID NO: 6 for the manufacture of a pharmaceutical preparation for preventing or attenuating an infection caused by a member of the genus *Streptococcus* in an animal.

## Patentansprüche

1. Impfstoff, umfassend als Wirkstoff ein Polypeptid, umfassend die Sequenz von SEQ ID NO: 6.

2. Impfstoffzusammensetzung nach Anspruch 1 zusammen mit einem pharmazeutisch verträglichen Träger; wobei das Polypeptid in einer Menge vorhanden ist, die in einem Tier wirksam Antikörper gegen einen Organismus der Gattung *Streptococcus* hervorruft.

3. Impfstoff nach Anspruch 1, wobei das Polypeptid durch einen mikrobiellen Organismus, transformiert mit Polynucleotiden und **dadurch** exprimierend das Polypeptid von SEQ ID NO: 6, als einen Wirkstoff des Impfstoffs, bereitgestellt wird.

4. Impfstoff nach Anspruch 3 zur Anwendung beim Verhindern oder Abschwächen einer Infektion, verursacht durch ein Mitglied der Gattung *Streptococcus,* bei einem Tier.

5. Impfstoff nach Anspruch 3, wobei der transformierte Mikroorganismus aus der Gruppe, bestehend aus Salmonellen, Mykobakterien, Streptokokken, Pockenviren und Adenoviren, ausgewählt ist.

6. Verwendung eines Polypeptids, umfassend die Sequenz von SEQ ID NO: 6 für die Herstellung einer pharmazeutischen Zubereitung zum Verhindern oder Abschwächen einer Infektion, verursacht durch ein Mitglied der Gattung *Streptococcus,* bei einem Tier.

7. Verwendung eines Antikörpers, der sich spezifisch an ein Polypeptid bindet, umfassend die Sequenz von SEQ ID NO: 6 für die Herstellung einer pharmazeutischen Zubereitung zum Verhindern oder Abschwächen einer Infektion, verursacht durch ein Mitglied der Gattung *Streptococcus,* bei einem Tier.

## Revendications

1. Vaccin comprenant un polypeptide en tant que principe actif, comprenant la séquence de SEQ ID NO: 6.

2. Composition de vaccin selon la revendication 1 ainsi qu'un excipient pharmaceutiquement acceptable, où le polypeptide est présent en une quantité efficace pour obtenir des anticorps contre un organisme du genre *Streptococcus* chez un animal.

3. Vaccin selon la revendication 1, dans lequel le polypeptide est fourni par un organisme microbien transformé avec des polynucléotides et exprimant ainsi le polypeptide de la SEQ ID NO:6, en tant que principe actif dudit vaccin.

4. Vaccin selon la revendication 3 en vue d'une utilisation dans la prévention ou l'atténuation d'une infection causée par un membre du genre *Streptococcus* chez un animal.

5. Vaccin selon la revendication 3, dans lequel ledit micro-organisme transformé est sélectionné parmi le groupe consistant en salmonelles, mycobactéries, streptocoques, poxvirus et adénovirus.

6. Utilisation d'un polypeptide comprenant la séquence de SEQ ID NO:6 pour la fabrication d'une préparation pharmaceutique destinée à prévenir ou à atténuer une infection causée par un membre du genre *Streptococcus* chez un animal.

7. Utilisation d'un anticorps qui se lie spécifiquement à un polypeptide comprenant la séquence de SEQ ID NO:6 pour la fabrication d'une préparation pharmaceutique destinée à prévenir ou à atténuer une infection causée par un membre du genre *Streptococcus* chez un animal.
